# EUROPÄISCHE PATENTANMELDUNG

(11) **EP 0 779 290 A1**
(43) Veröffentlichungstag der Anmeldung: **18.06.1997**
(21) Anmeldenummer: 96118449.6
(22) Anmeldetag: 18.11.1996
(51) Int. Cl.: C07D 317/36, A61K 6/09, G03F 7/027, C08F 2/46

(54) **Urethan-(Meth)acrylate mit cyclischen Carbonatgruppen und deren Verwendung zur Herstellung von Dentalwerkstoffen oder lithographischen Druckplatten**

(30) Priorität: 30.11.1995 DE 19544671
(71) Anmelder: BAYER AG, 51368 Leverkusen (DE)
(72) Erfinder: Podszun, Wolfgang, Dr., 51061 Köln (DE); Krüger, Joachim, 40789 Monheim (DE); Finger, Werner, Prof. Dr., 41469 Neuss (DE); Heiliger, Ludger, Dr., 51373 Leverkusen (DE); Casser, Carl, Dr., 51061 Köln (DE)

(57) **Zusammenfassung**

Die Erfindung betrifft Urethanacrylate und Urethanmethacrylate mit cyclischen Carbonatgruppen gemäß Formel III 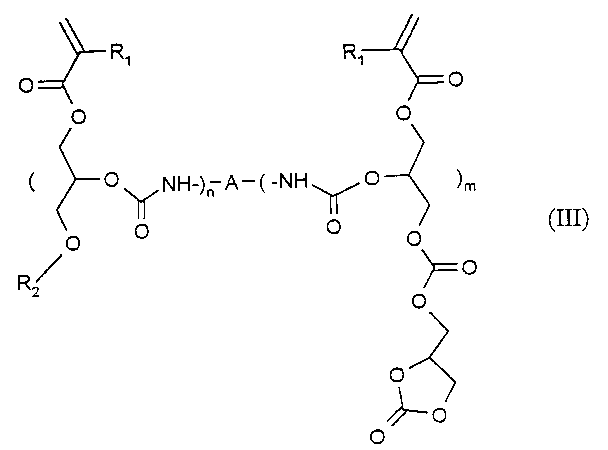 worin
- R₁: für Wasserstoff oder Methyl,
- R₂: für Wasserstoff oder (Meth)acryl steht,
- A: für einen n- und m-wertigen Kohlenwasserstoffrest mit 2 bis 30 Kohlenstoffatomen, der mit einer oder mehreren Ether-, Ester-, Amid-, Urethan- oder Harnstoffgruppen unterbrochen sein kann und mit ein bis fünf (Meth)acrylatgruppen substituiert sein kann, steht
- n: eine ganze Zahl von 0 bis 3 und
- m: eine ganze Zahl von 1 bis 4 bedeutet,
sowie ihre Verwendung in Dentalwerkstoffen und lithografischen Druckplatten.

## Beschreibung

Die Erfindung betrifft Urethanacrylate und Urethanmethacrylate mit cyclischen Carbonatgruppen, sowie ihre Verwendung in Dentalwerkstoffen.

Urethan(meth)acrylate werden beispielsweise für Beschichtungen, in Klebstoffen und auf dem Dentalgebiet breit angewendet. Ein auf dem Dentalgebiet besonders häufig verwendetes Monomer ist das Umsetzungsprodukt aus 2,2,4-Trimethylhexamethylendiisocyanat und 2-Hydroxyethylmethacrylat entsprechend Formel (I).

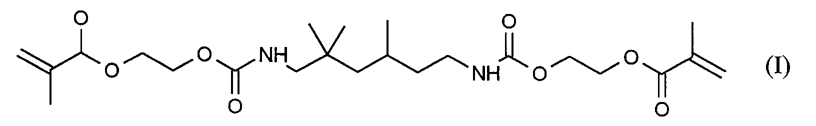

Verbindung (I) wird z.B. als Bestandteil von Kunststoffüllungsmaterialien, Zahnlacken, Versieglern, Befestigungsmaterialen und Verblendmaterialien eingesetzt.

In der US-A-4 400 159 werden Addukte von 3-Methacryloyl-2-hydroxypropylestern mit Diisocyanaten als Monomere für Dentalmaterialien vorgeschlagen. Diese Monomerklasse entspricht der allgemeinen Formel (II), 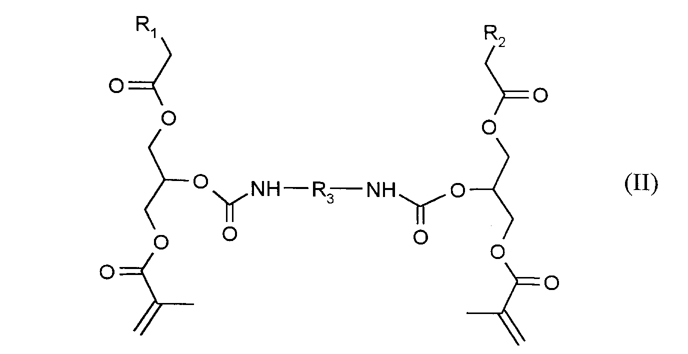 worin R₁ und R₂ beispielsweise eine Cyclohexyl- oder Phenylgruppe und R₃ ein aliphatischer, cycloaliphatischer oder aromatischer Rest mit 6 bis 14 Kohlenstoffatomen bedeuten kann.

Werkstoffe aus Urethan(meth)acrylaten weisen ein günstiges Niveau der mechanischen Eigenschaften auf. Eine bevorzugte Aushärtungsmethode für viele Anwendungen ist die Photopolymerisation unter Bestrahlung mit sichtbarem Licht. Für die Aushärtung von Zahnfüllungsmaterialien beträgt die notwendige Belichtungsdauer üblicher Weise 15 bis 60 s. Die Aushärtungstiefen betragen im allgemeinen einige mm und sind bei stark pigmentierten Füllungsmaterialien deutlich niedriger als bei transparenten Füllungsmaterialien. Bei tiefen Kavitäten muß der Zahnarzt die Füllung schichtweise aushärten.

Ein Mangel aller bisher bekannten photopolymerisierbaren Dentalmaterialien besteht darin, daß sie in Kontakt mit Luftsauerstoff an der freien Oberfläche nicht aushärten. Dieses als Polymerisationsinhibition bekannte Phänomen erfordert besondere Maßnahmen zum Ausschluß des Luftsauerstoffs. Eine gut wirksame Maßnahme bei glatten Oberflächen ist die Abdeckung mit einer transparenten sauerstoffundurchlässigen Folie. Für geometrisch kompliziertere Oberflächen wurden Schutzüberzüge (z.B. aus Polyvinylalkohol), die aus Lösung aufgetragen werden, als Sauerstoffbarriere vorgeschlagen, doch ist diese Methode nicht nur aufwendig sondern auch weniger wirksam. In vielen Fällen behilft sich der Zahnarzt in der Weise, daß er einen Überschuß an Dentalmaterial anwendet und den nicht polymerisierten Teil nach der Belichtung in einem Nachbearbeitungsschritt entfernt. Auch diese Methode birgt gravierende Nachteile: Die Gestaltung der Oberflächengeometrie ist nur sehr ungenau möglich. Die Grenze zwischen nicht oder unzureichend polymerisierten Material einerseits und vollständig ausgehärteten Material andererseits ist nicht erkennbar, sodaß die Gefahr besteht, daß bei der Nachbearbeitung eine nicht optimal ausgehärtete Oberfläche erhalten wird.

Die Aufgabe der vorliegenden Erfindung ist die Bereitstellung neuer Monomere mit erhöhter Polymerisationsgeschwindigkeit und verringerter Empfindlichkeit gegenüber Polymerisationshemmung durch Sauerstoff, insbesondere für Anwendungen auf dem Dentalgebiet.

Die erfindungsgemäße Aufgabe wird gelöst durch Monomere mit cyclischen Carbonatgruppen gemäß Formel III 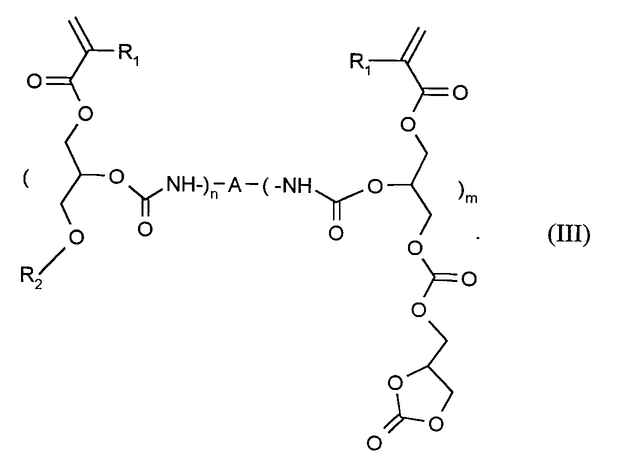 worin
- R₁: für Wasserstoff oder Methyl,
- R₂: für Wasserstoff oder (Meth)acryl steht,
- A: für einen n- und m-wertigen Kohlenwasserstoffrest mit 2 bis 30 Kohlenstoffatomen, der mit einer oder mehreren Ether-, Ester-, Amid-, Urethan- oder Harnstoffgruppen unterbrochen sein kann und mit ein bis fünf (Meth)acrylatgruppen substituiert sein kann, steht
- n: eine ganze Zahl von 0 bis 3
und
- m: eine ganze Zahl von 1 bis 4 bedeutet.

Bevorzugt werden Monomere gemäß Formel IIIa 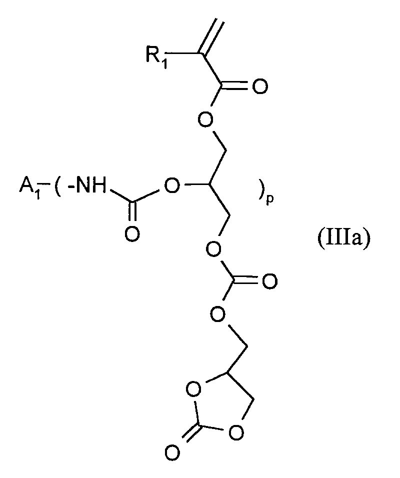 worin
- R₁: für Wasserstoff oder Methyl,
- A₁: für einen p-wertigen Kohlenwasserstoffrest mit 2 bis 30 Kohlenstoffatomen, der mit einer oder mehreren Ether-, Ester-, Amid-, Urethan- oder Harnstoffgruppen unterbrochen sein kann,
- p: 2 oder 3 bedeutet.

Die erfindungsgemäßen Monomeren können in unterschiedlichen stereoisomeren Strukturen vorliegen, wobei nur ein Teil der möglichen Strukturen durch die Formel (III) direkt erfaßt wird. Durch die Formel (IIIb) wird beispielsweise ein Stereoisomeres dargestellt. Die erfindungsgemäßen Monomere betreffen alle möglichen Steroisomere und Kombinationen davon.

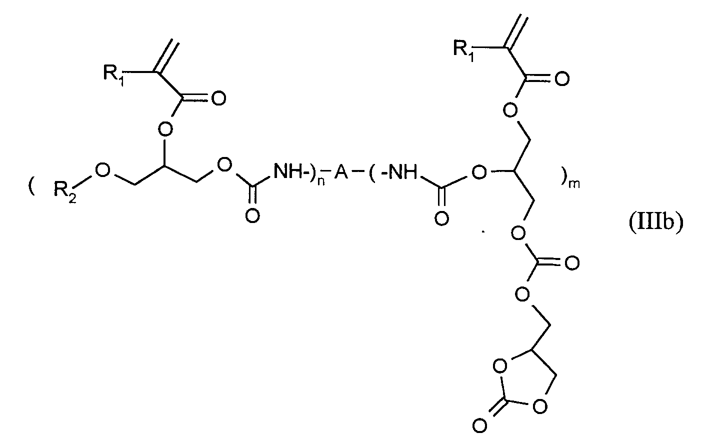

Als geeignete Kohlenwasserstoffreste A bzw. A₁ seien genannt:

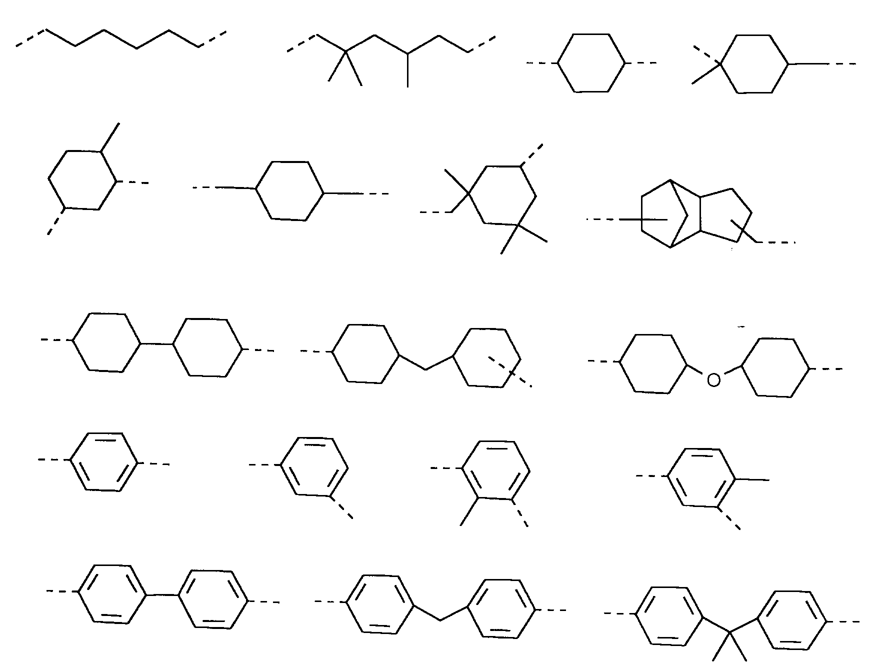

### Tabelle 1 erfindungsgemäße Monomere

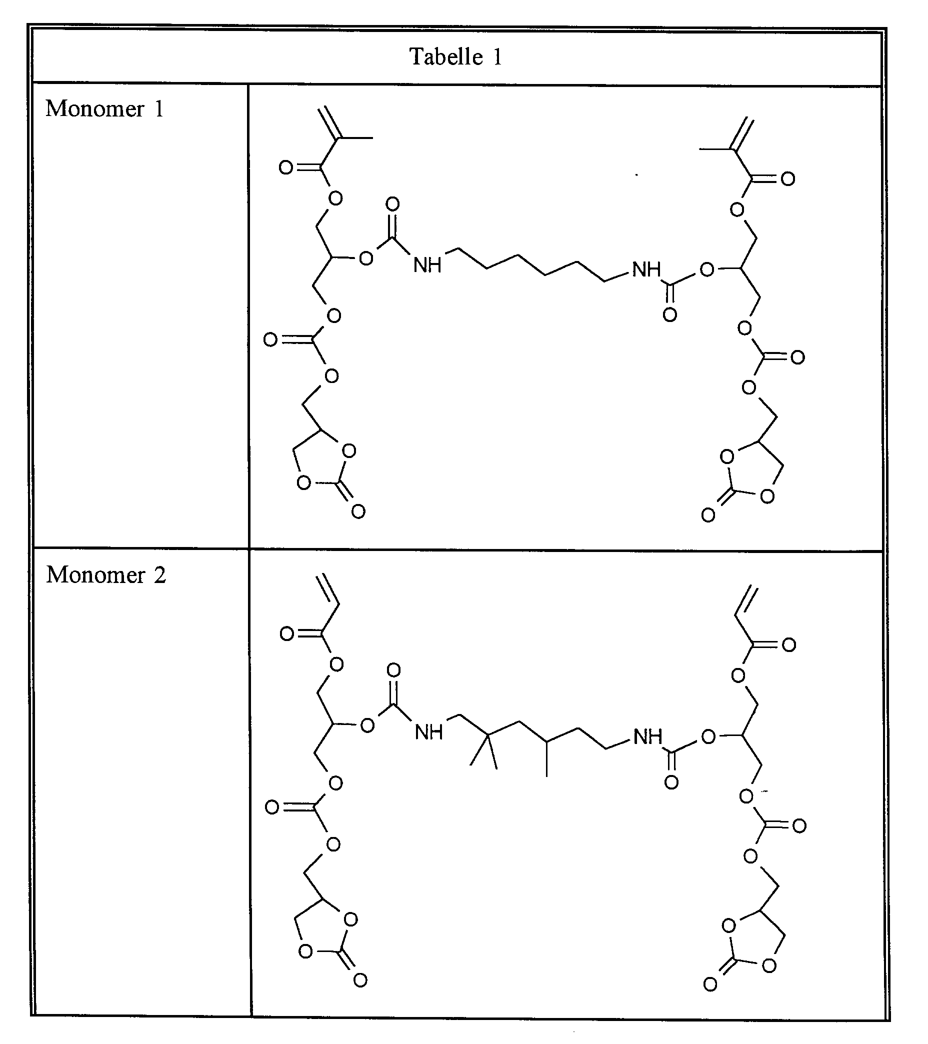

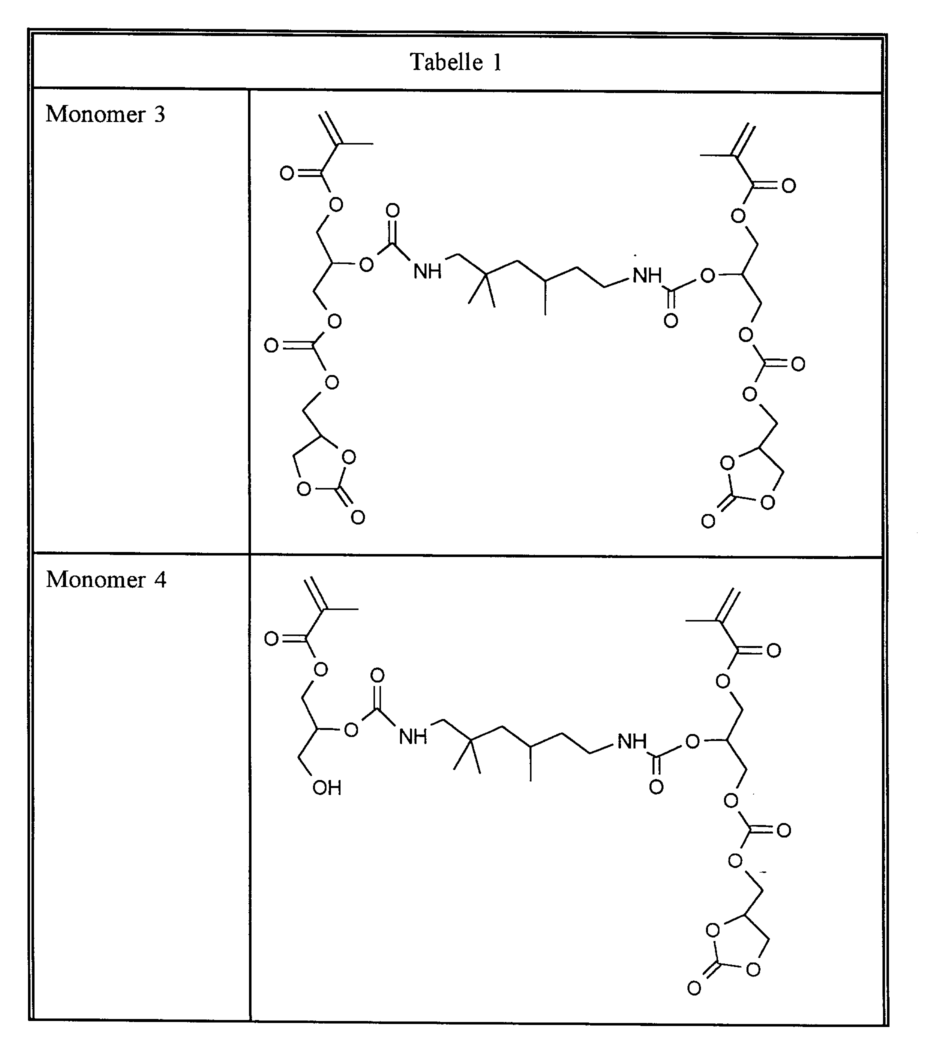

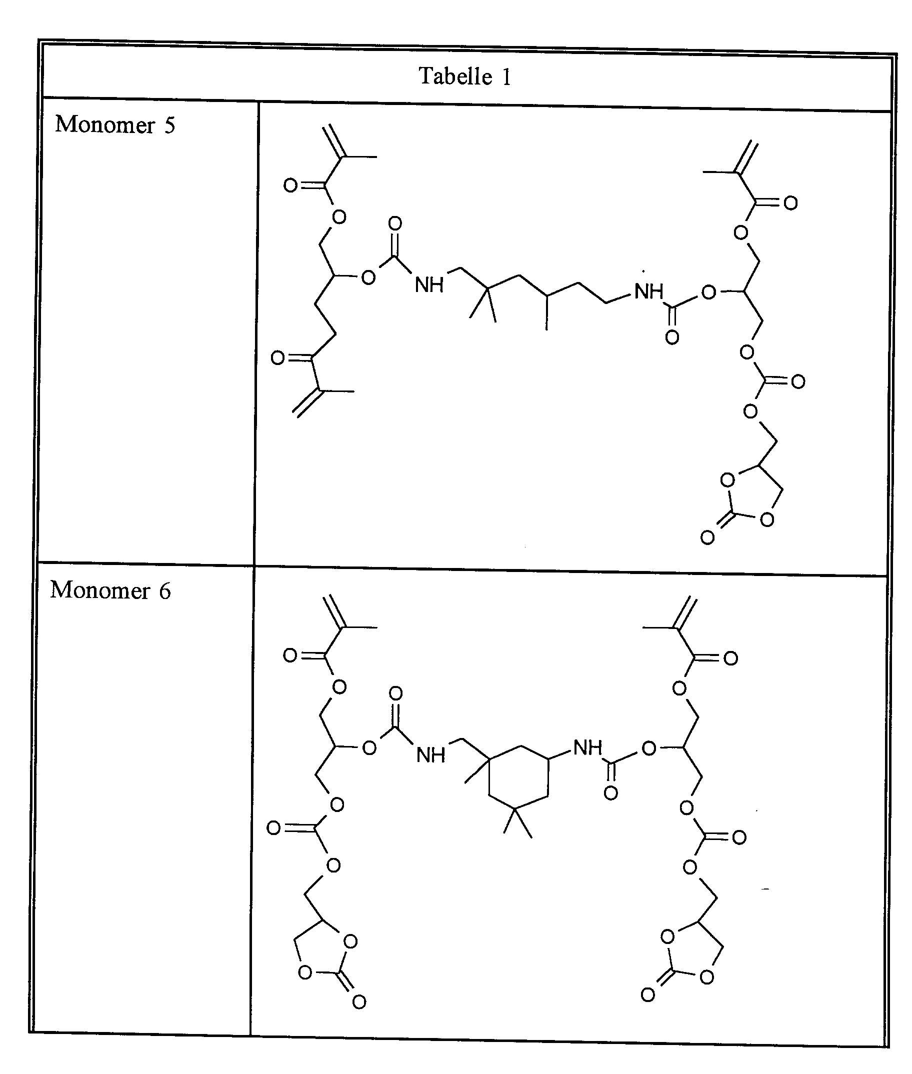

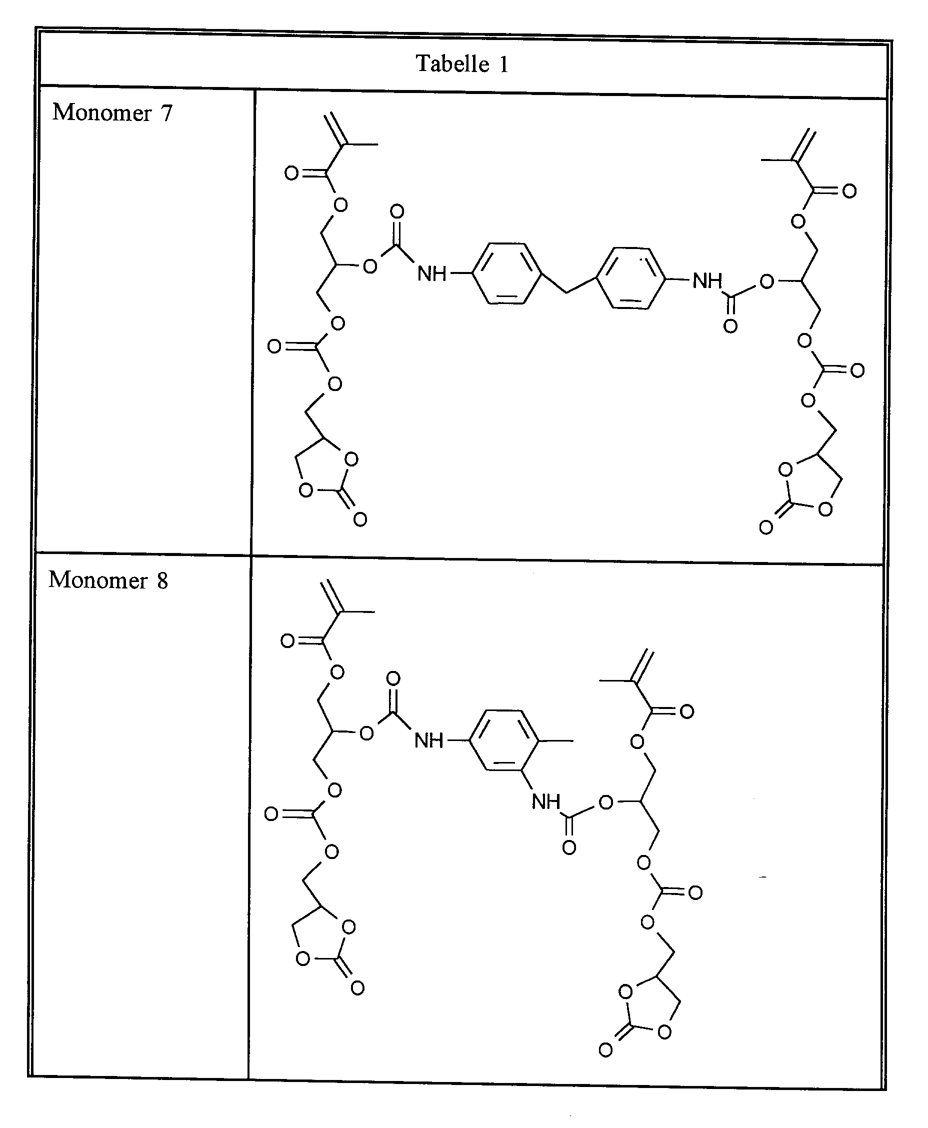

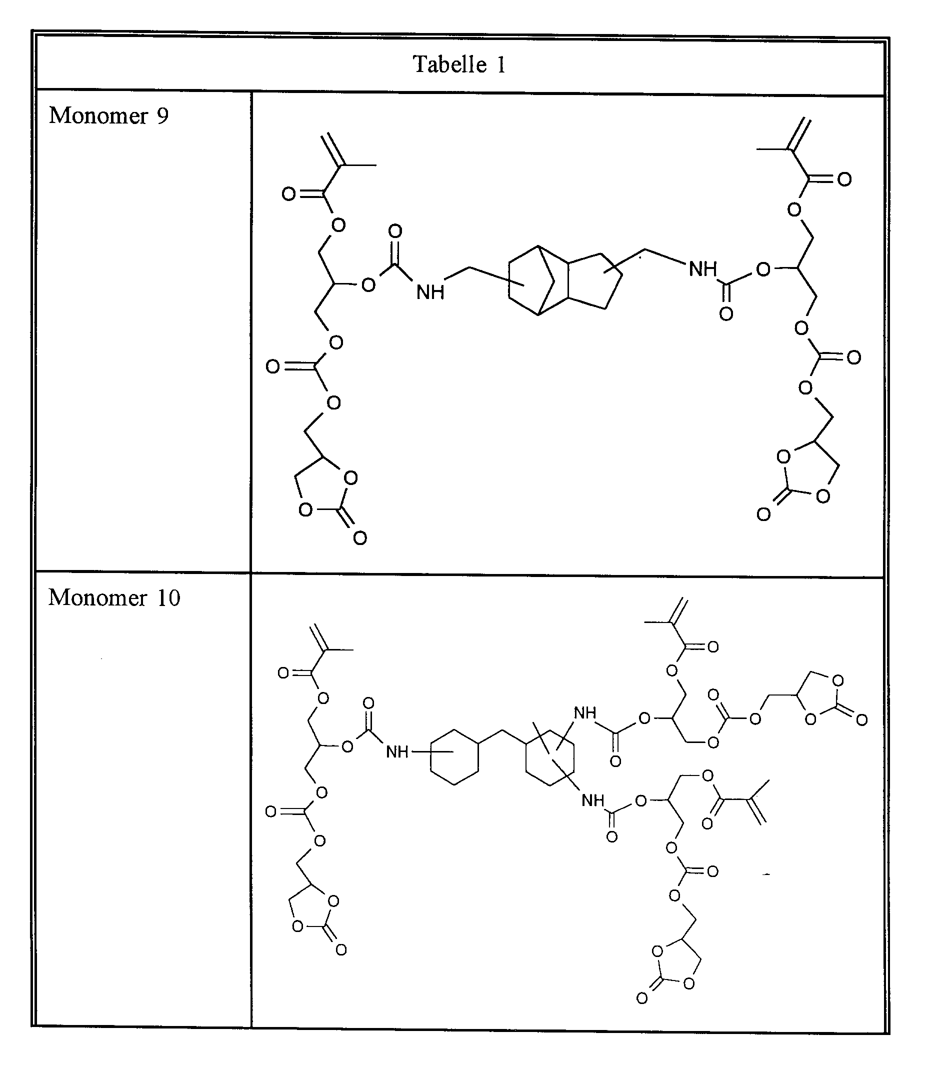

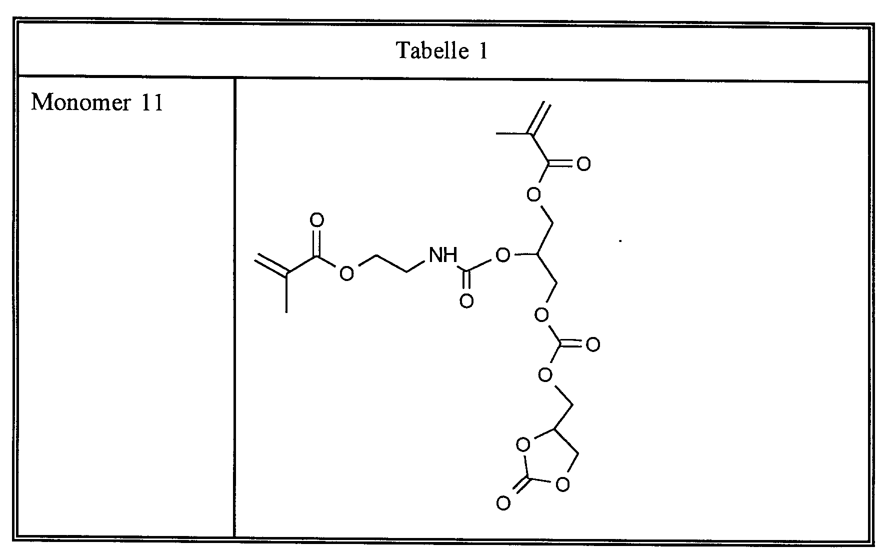

Die Synthese der erfindungsgemäßen Urethan(meth)acrylate mit cyclischen Carbonatgruppen erfolgt zweckmäßiger Weise durch stöchiometrische Umsetzung der Hydroxylverbindung gemäß Formel IV, gegebenenfalls im Gemisch mit weiteren hydroxyfunktionellen (Meth)acrylester, mit einem Mono-, Di-, Tri-oder Tetraisocyanat.

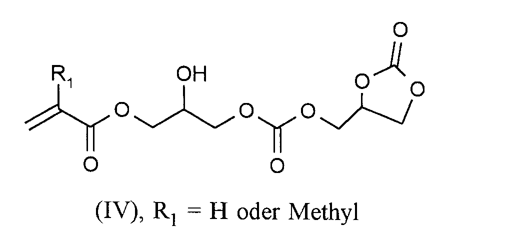

Die genannte Umsetzung wird vorzugsweise in einem inerten Lösungsmittel in Anwesenheit eines Katalysators bei Temperaturen- von z.B. -20 °C bis 60 °C durchgeführt. Als inertes Lösungsmittel seien beispielhaft Aceton, Butanon-2, Tetrahydrofuran, Methylenchlorid, Chloroform, Toluol und Acetonitril genannt. Als Katalysatoren können Metallsalz höherer Fettsäuren, wie z.B. Dibutylzinnlaurat, Triarylverbindungen, wie z.B. Triphenylstibin oder Triphenylphosphin, oder tertiäre Amine, wie z.B. Triethylamin eingesetzt werden.

Die Hydroxyverbindung nach Formel IV ist beispielsweise durch Umsetzung von Glycerinmono(meth)acrylat mit dem Chlorameisensäureester gemäß Formel V zugänglich. Der Chlorameisensäureester gemäß Formel V kann durch Phosgenierung von Glycerin hergestellt werden. Dieser Syntheseschritt wird in US-A-2 446 145 ausführlich beschrieben.

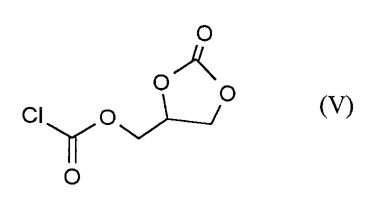

Die Hydroxyverbindung IV kann auch durch Umsetzung von Glycerol mit dem Säurechlorid (V) und anschließender Öffnung des Epoxidrings mit (Meth)acrylsäure synthetisiert werden. Nach dieser Verfahrensvariante ist Verbindung IV in besonders hoher Reinheit erhältlich.

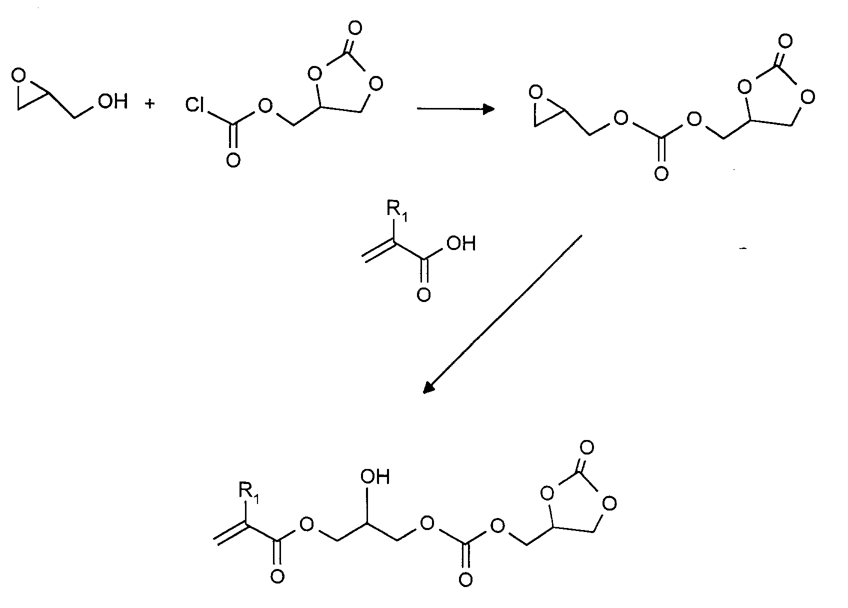

Für die Anwendung der erfindungsgemäßen Monomere in polymerisierbaren dentalen Restaurierungsmaterialien können die erfindungsgemäßen Urethan(meth)acrylate mit an sich bekannten Monomeren gemischt werden. Als Mischungskomponente sind beispielsweise das sogenannte BisGMA und Urethanmethacrylate, beispielsweise die Verbindung nach Formel I, geeignet.

Die Viskosität der erfindungsgemäßen Monomeren ist für viele Anwendungen geeignet. Falls eine noch niedrigere Viskosität gewünscht wird, können den erfindungsgemäßen Monomeren Comonomere niedriger Viskosität als Reaktivverdünner bzw. Lösungsmittel zugemischt werden. Die erfindungsgemäßen Verbindungen werden in der Mischung mit Comonomeren mit einem Anteil von ca. 10 bis 100 Gew.-%, bevorzugt von 20 bis 80 Gew.-%, eingesetzt.

Beispielsweise seien die folgenden Comonomere genannt: Glycerindimethacrylat, Triethylenglykoldimethacrylat, (TEGDMA), Tetraethylenglykoldimethacrylat, 1,12-Dodecandioldimethacrylat, 1,6-Hexandioldimethacrylat, Diethylenglykoldimethacrylat, 2,2-Bis-[p-(2'-hydroxy-3'-methacryloyloxypropoxy)-phenyl]-propan, 2,2-Bis-[p-(2'-methacryloyloxyethoxy)-phenyl]-propan, Trimmethylol-propan-tri-(meth)-acrylat, Bis-(meth)-acryloyloxyethoxymethyl-tricyclo-[5,5,1,0^{2,6}]-decan (DE-A 2 931 925 und DE-A 2 931 926).

Insbesondere bevorzugt werden Comonomere, die bei 13 mbar einen Siedepunkt über 100°C besitzen.

Die erfindungsgemäßen (Meth)acrylsäureester können gegebenenfalls in Mischung mit den genannten Comonomeren mit an sich bekannten Methoden zu vernetzten Polymerisaten aushärten (Am. Chem. Soc. Symp. Ser. 212, 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen.

Die peroxid- bzw. aminhaltigen Monomermischungen werden bis zur Anwendung getrennt gelagert.

Die erfindungsgemäßen Monomeren können durch Bestrahlung mit UV-Licht oder sichtbarem Licht (beispielsweise im Wellenlängenbereich von 230 bis 650 nm) zur Polymerisation gebracht werden. Als Initiatoren für die photoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von syergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE-PS 3 135 115 beschrieben.

Neben den oben beschriebenen Initiatoren können den erfindungsgemäßen Monomeren an sich für diesen Einsatzzweck bekannte Lichtschutzmittel und Stabilisatoren zugesetzt werden.

Lichtschutzmittel sind beispielsweise in "Gächter, Müller, Taschenbuch der Kunststoff-Additive, 2. Ausgabe, Carl Hanser Verlag" beschrieben. Beispielsweise seien die folgenden Lichtschutzmittel genannt: Cyasorb UV 9®, Tinuvin P®, Tinuvin 770®, Tinuvin 622®, Tinuvin 765®.

Stabilisatoren sind beispielsweise in "Ullmanns Encyclopädie der technischen Chemie, 4. Auflage, Band 8" beschrieben. Beispielsweise seien die folgenden Stabilisatoren genannt:
2,6-Di-tert.-butylphenol, 2,6-Di-tert.-butyl-4-methylphenol, 2,6-Di-octadecyl-4-methyl-phenol, 1,1'-Methylen-bis(naphthol-2) u.a..

Die Lichtschutzmittel und die Stabilisatoren können jeweils in einer Menge von 0,01 bis 0,5 Gew.-Teilen, bezogen auf 100 Gew.-Teile der Monomermischung, eingesetzt werden.

Die Monomermischungen können mit oder ohne Zusatz von Füllstoffen auch als Beschichtungsmittel (Zahnlacke) und als Adhäsive (Schmelz und Dentin) eingesetzt werden.

Bei der Verwendung als Zahnfüllmassen oder Befestigungsmaterialien setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPa.s besitzen, besonders vorteilhaft.

Vorzugsweise mischt man den erfindungsgemäßen Monomeren anorganische Füllstoffe zu. Beispielsweise seien Bergkristall, Kristobalit, Quarzglas, hochdisperse Kieselsäure, Aluminiumoxid und Glaskeramiken, beispielsweise Zirkon enthaltende Glaskeramiken (DE-OS 2 347 591) genannt, Die anorganischen Füllstoffe werden zur Verbesserung des Verbunds zur Polymermatrix des Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297-308 (1983), Bevorzugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen mittleren Teilchendurchmesser und/oder einen unterschiedlichen Silangehalt besitzen.

Der Füllstoffanteil in der Zahnfüll- wie in der Befestigungsmasse beträgt im allgemeinen 5 bis 85 Gew.-%, vorzugsweise 50 bis 80 Gew.-%.

Für die Herstellung der Restaurierungsmassen werden die Komponenten unter Verwendung handelsüblicher Knetmaschinen vermischt.

Der Anteil der erfindungsgemäßen (Meth)acrylate in den Restaurierungsmassen beträgt im allgemeinen 5 bis 60 Gew.-%, bezogen auf die Restuarierungsmasse.

Zahnlacke, Adhäsive und Restaurationsmaterialien, die die erfindungsgemäßen Monomere enthalten, zeigen eine äußerst hohe Polymerisationsgeschwindigkeit, wobei die Polymerisation nur wenig durch Luftsauerstoff gestört wird. Bei der Photopolymerisation werden große Aushärtungstiefen erreicht.

Zur Prüfung der Empfindlichkeit erfindungsgemäßer Monomere gegenüber Polymerisationsinhibition durch Luftsauerstoff wurde die unpolymerisierte Schichtdicke durch Messung der Probenoberfläche vor und nach Auflösung der Monomerschicht in einem geeigneten Lösungsmittel bestimmt.

Die erfindungsgemäßen Monomere können darüber hinaus zur Herstellung von lithografischen Druckplatten verwendet werden.

### Beispiele

### Beispiel 1

### Herstellung von Monomer 1 aus Tabelle 1

In 300 g Chloroform wurden unter Stickstoffatmosphäre 32,0 g Glycerinmonomethacrylat 32,38 g Triethylamin, 0,08 g 2,6-Di-tert.-butylkresol (Stabilisator) und 0,08 g Dibutylzinndilaurat (Katalysator) gelöst und auf -10 °C gekühlt. Danach wurden 54,17 g Chlorameisensäureester der Formel III, gelöst in 100 g Chloroform innerhalb von 30 min zudosiert und der Ansatz noch 20 h bei Raumtemperatur nachgerührt. Danach wurden 16,8 g Hexamethylendiisocyanat innerhalb von 30 min zugetropft. Es wurde solange nachgerührt (ca. 10 h) bis im IR-Spektrum keine NCO-Bande nachweisbar war. Der entstandene Niederschlag wurde abfiltriert und das Filtrat auf das doppelte Volumen Wasser gegossen. Die organische Phase wurde abgetrennt, 4 mal mit Wasser gewaschen und über Natriumsulfat getrocknet. Nach Filtration und Abdampfen des Lösungsmittels verbleiben 64,6 g Monomer 1 aus Tabelle 1.
IR [cm⁻¹]: 1 800 (cycl. Carbonat); 1 720 (Urethan); 1 640 (Methacryl).

### Beispiel 2

### Herstellung der Monomeren 3, 6, 8, 9, 10 und 11 aus Tabelle 1

Die Versuche wurden ausgeführt wie in Beispiel 1 beschrieben, mit der Änderung, daß anstelle von 16,8 g Hexamethylendiisocyanat die unten aufgeführten Monomere in den angegebenen Mengen eingesetzt wurden.

| **Monomer aus Tabelle 1** | **Isocyanat** | **Einsatzmenge** |
|---|---|---|
| Monomer 3 | 2,2,4-Trimethylhexamethylendiisocyanat | 21,0 g |
| Monomer 6 | Isophorondiisocyanat | 22,2 g |
| Monomer 8 | 1,3-Tolylendiisocyanat | 17,4 g |
| Monomer 9 | Bisisocyanatomethyltricyclo-(5.2.1.)-decan | 24,6 g |
| Monomer 10 | Triisocyanato-methyl-dicyclohexylmethan (Isomerengemisch mit einem NCO-Gehalt von 37 Gew.-%) | 21,2 g |
| Monomer 11 | 2-Isocyanatoethylmethacrylat | 31,0 g |

### Beispiel 3

### Überprüfung der Photoreaktivität der Monomeren mit Hilfe der Photo-DSC

Die folgenden Bestandteile wurden intensiv durchmischt:
- 5,0 g: Monomer
- 100 g: Campherchinon
- 250 mg: p-Dimethylaminobenzolsulfonsäure-N,N-diallylamid

Campherchinon und p-Dimethylaminobenzolsulfonsäure-N,N-diallylamid bilden das Photoinitiatorsystem.

Die Proben wurden bei 30 °C in einer DSC-Apparatur (Differential Scanning Calorimetry) mit einer Halogenlampe (75 W) mit Wärmeschutzfilter bestrahlt. Der Wärmefluß wurde unter Bestrahlung als Funktion der Zeit registriert. Als Referenz wurden Proben gleicher Zusammensetzung ohne Photoinitiator eingesetzt. Während des Versuchs wurde mit Stickstoff gespült. Für die Auswertung wurde als Maß für die Reaktionsgeschwindigkeit der Wert t-max ermittelt. t-max ist die Zeit von Bestrahlungsbeginn bis zum Erreichen des Maximums der Reaktion (maximaler Wärmefluß).
Je kleiner t-max ist, um so größer ist die Photoreaktivität.

| **Monomer aus Tabelle 1** | **t-max [min]** |
|---|---|
| Monomer 1 | 0,90 |
| Monomer 3 | 0,62 |
| Monomer 6 | 1,06 |
| Monomer 9 | 0,88 |
| Monomer 10 | 0,94 |
| Monomer 11 | 0,72 |
| Vergleich: Kommerzielles Monomer nach Formel I | 1,85 |
| Vergleich: Beispiel 2 der US 4 400 159 | 2,59 |

### Beispiel 4

### Untersuchung der Empfindlichkeit gegenüber Polymerisationsinhibition durch Luftsauerstoff

Zur Bestimmung der Schichtdicke unpolymerisierten Monomers an der freien Oberfläche (normale Umgebungsatmosphäre) wurden zylinderische Metallformen (Ø = 5 mm, h = 2 mm) mit lichtaktiviertem Monomer (Aktivierung wie in Beispiel 3) aufgefüllt und 20 Sekunden lang mit einer handelsüblichen Polymerisationseinheit (Translux® CL, Kulzer GmbH) im Abstand von ca. 2 mm belichtet. Unmittelbar nach der Polymerisation wurden im Auflichtmikroskop nach der Schärfentiefenmethode an jeweils 7 durch die Kreuztischkoordinaten definierten Meßpunkten (x, y) auf einer Geraden, unter Einschluß von Referenzpunkten auf der freien Metalloberfläche, die Höhenkoordinaten (z-Werte) bestimmt. Die Proben wurden dann zur Entfernung der nicht polymerisierten Oberflächenschicht unter Verwendung einer mittelharten Bürste mit Ethanol abgewaschen. Die Form wurde anschließend wieder in die Ausgangsposition auf dem Mikroskopkreuztisch montiert. Nach Anfahren der ursprünglichen x/y-Koordinaten wurden erneut die z-Werte bestimmt. Der Höhendifferenzwert vor und nach Abwaschen mit Alkohol entspricht der Monomerschichtdicke, die aufgrund der Sauerstoffinhibition der Polymerisation an der Oberfläche verblieben war.

Die Monomerschichtdicken wurden an fünf Proben bestimmt und sind als Mittelwerte und Standardabweichungen in der folgenden Tabelle zusammengestellt:

| **Monomer aus Tabelle 1** | **Schichtdicke des unpolymerisierten Monomers [µm]** |
|---|---|
| Monomer 3 | 1,3 ± 0,9 |
| Monomer 6 | 0,9 ± 1,5 |
| Monomer 8 | 2,0 ± 1,5 |
| Monomer 9 | 0,0 ± 1,5 |
| Monomer 10 | 1,0 ± 1,2 |
| Vergleich: Kommerzielles Monomer nach Formel I | 9,8 ± 2,8 |
| Vergleich: Beispiel 2 der US 4 400 159 | 3,2 ± 1,4 |

Nach dem Abbürsten mit Ethanol zeigten die Vergleichsproben ausgeprägte Abriebsspuren, während Bürstenabriebsspuren der Proben aus den Monomeren der Beispiele 1 und 2 bei 200-facher Vergrößerung im Auflichtmikroskop kaum er erkennen waren. Diese Beobachtung belegt eine bessere oberflächliche Polymerisation der Proben aus den Monomeren der Beispiele 1 und 2 im Vergleich zur Referenzprobe.

## Patentansprüche

1. Monomere mit cyclischen Carbonatgruppen gemäß Formel III 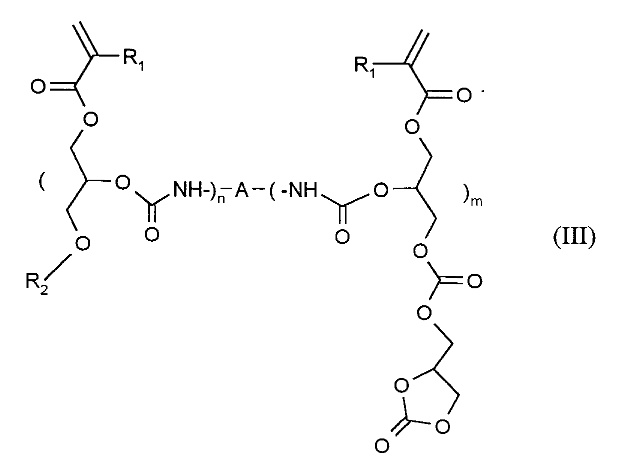 worin
R₁ für Wasserstoff oder Methyl,
R₂ für Wasserstoff oder (Meth)acryl steht,
A für einen n- und m-wertigen Kohlenwasserstoffrest mit 2 bis 30 Kohlenstoffatomen, der mit einer oder mehreren Ether-, Ester-, Amid-, Urethan- oder Harnstoffgruppen unterbrochen sein kann und mit ein bis fünf (Meth)acrylatgruppen substituiert sein kann, steht
n eine ganze Zahl von 0 bis 3
und
m eine ganze Zahl von 1 bis 4 bedeutet.

2. Monomere mit cyclischen Carbonatgruppen gemäß Anspruch 1 der Formel IIIa 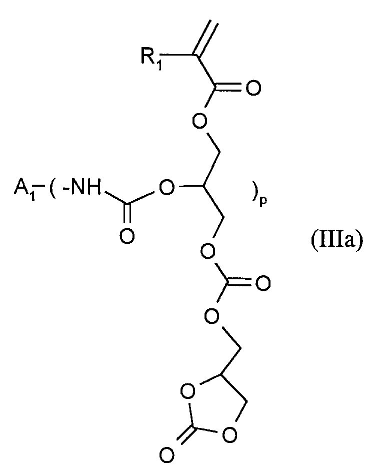 worin
R₁ für Wasserstoff oder Methyl,
A₁ für einen p-wertigen Kohlenwasserstoffrest mit 2 bis 30 Kohlenstoffatomen, der mit einer oder mehreren Ether-, Ester-, Amid-, Urethan- oder Harnstoffgruppen unterbrochen sein kann,
p 2 oder 3 bedeutet.

3. Monomere gemäß Anspruch 1 ausgewählt aus der Gruppe bestehend aus

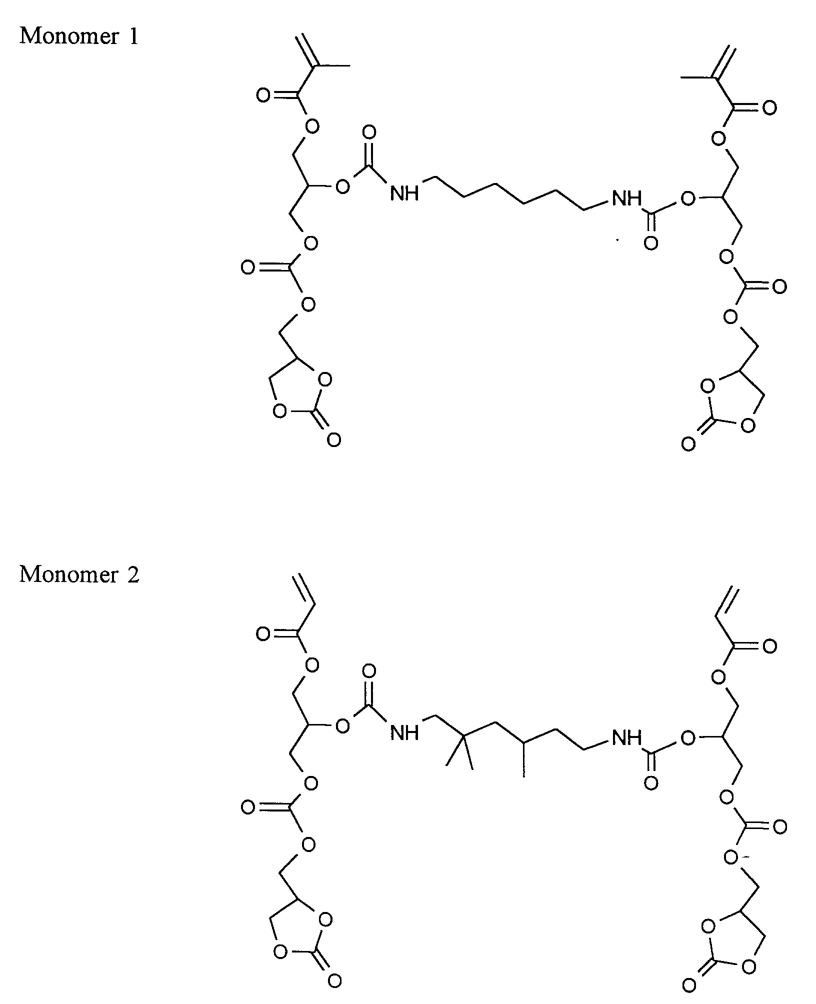

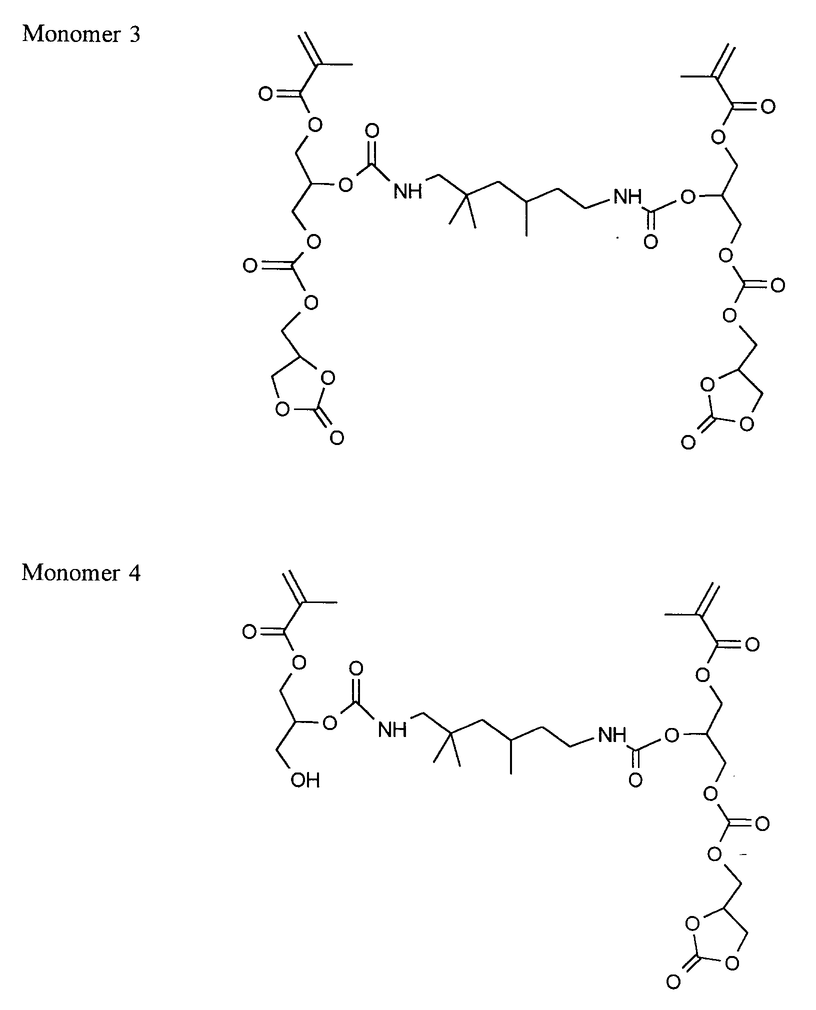

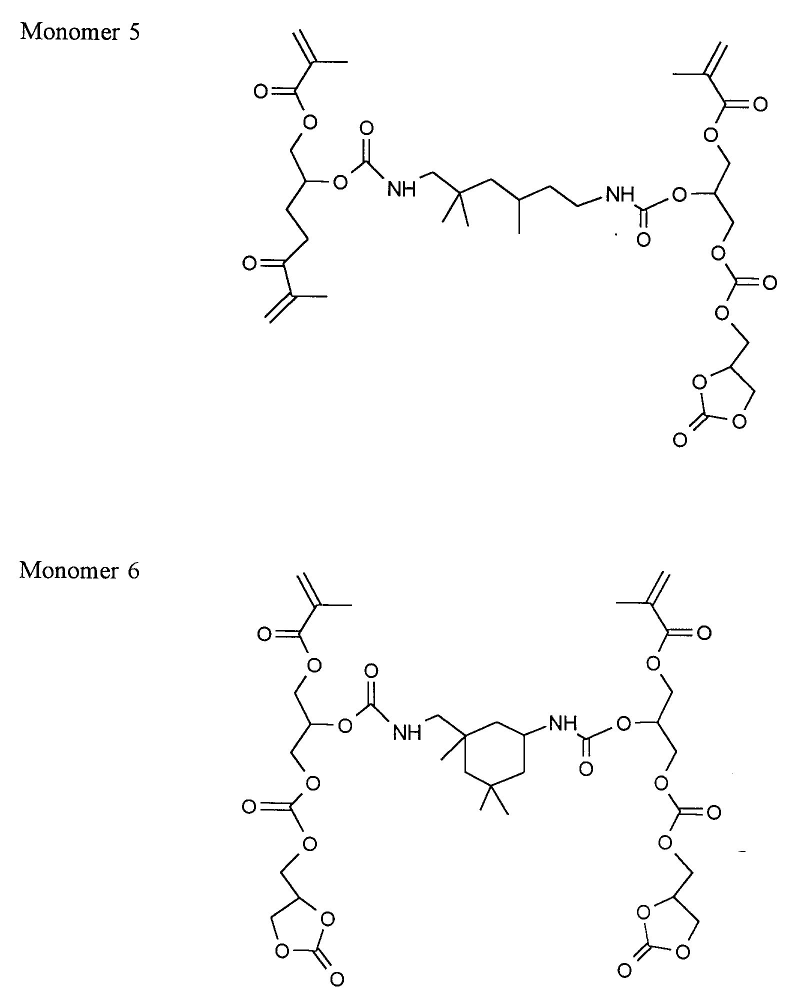

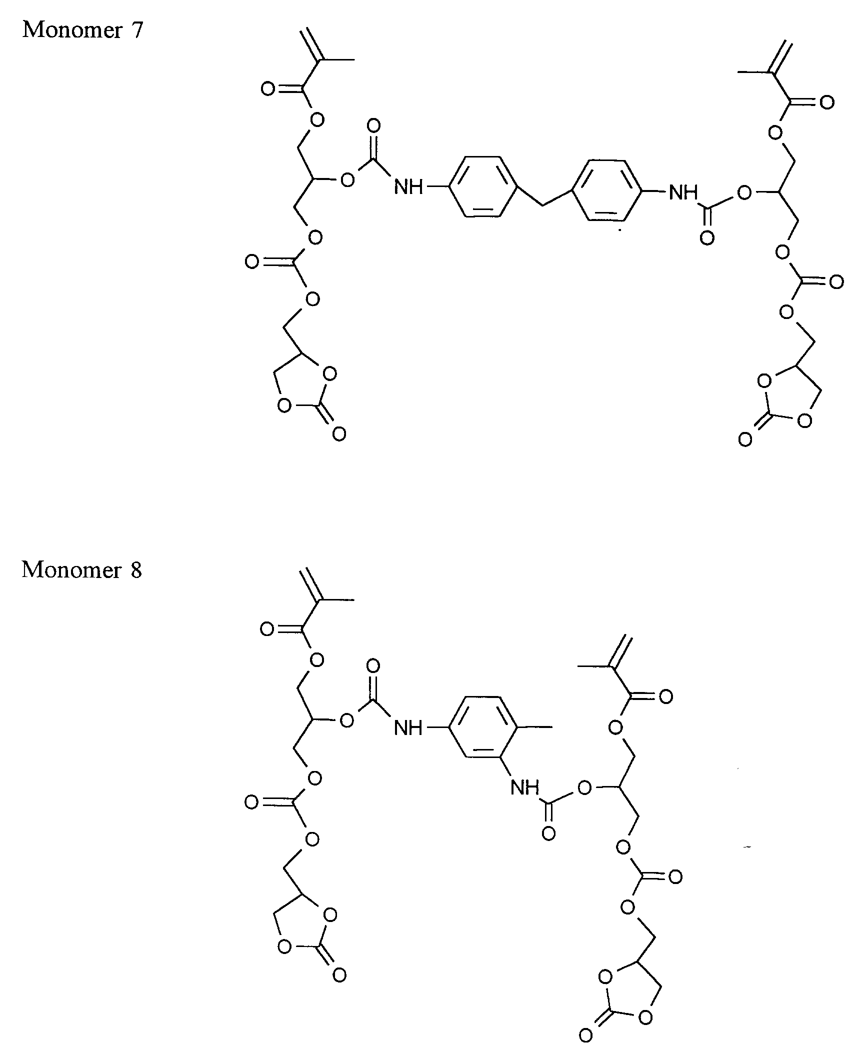

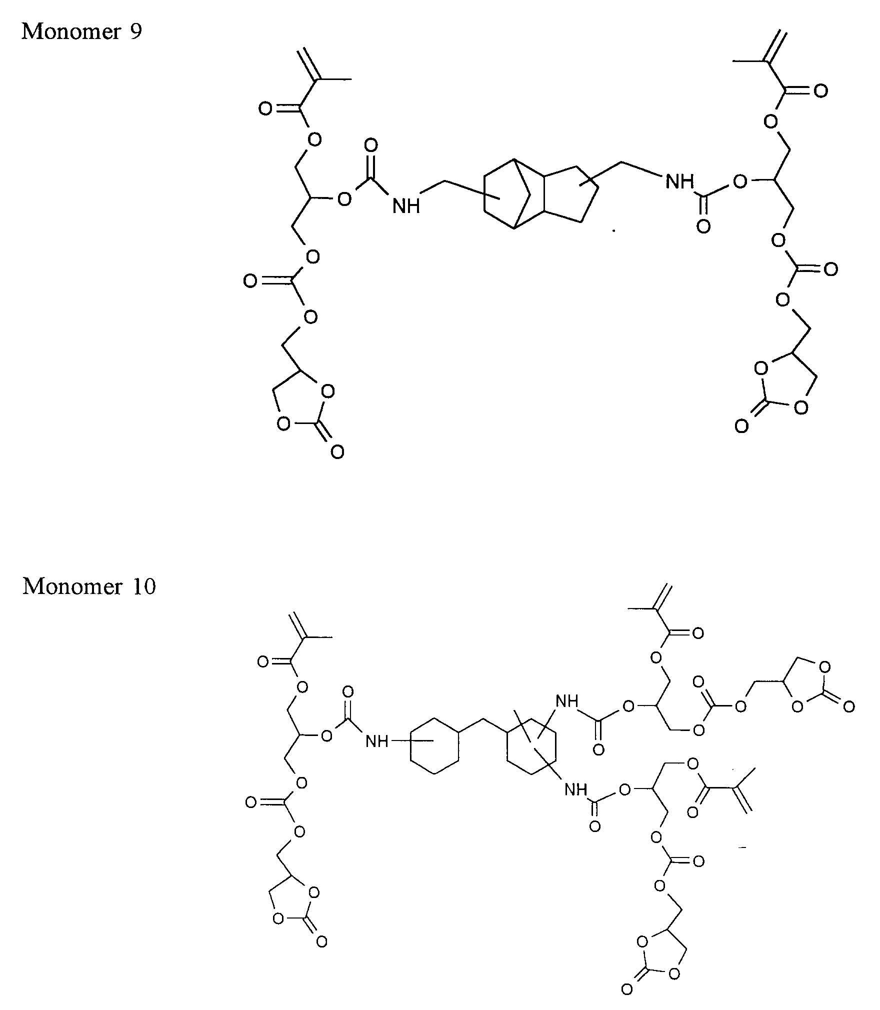

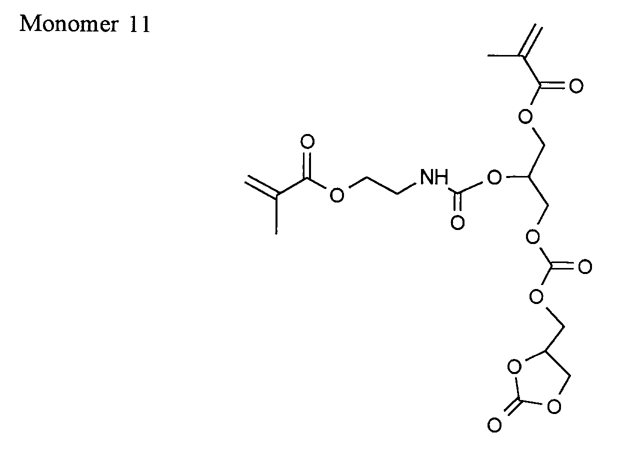

4. Zubereitungen enthaltend Monomere gemäß Ansprüchen 1 bis 3 und gegebenenfalls Comonomere sowie übliche Füll- und Hilfsstoffe.

5. Verwendung von Monomeren gemäß Ansprüchen 1 bis 3 zur Herstellung von Dentalwerkstoffen.

6. Verwendung von Monomeren gemäß Ansprüchen 1 bis 3 zur Herstellung von Adhäsiven.

7. Verwendung von Monomeren gemäß Ansprüchen 1 bis 3 zur Herstellung von Zahnlacken.

8. Verwendung von Monomeren gemäß Ansprüchen 1 bis 3 zur Herstellung von Restaurationsmaterialien.

9. Verwendung von Monomeren gemäß Ansprüchen 1 bis 3 zur Herstellung von lithografischen Druckplatten.
